# EUROPEAN PATENT SPECIFICATION

(11) **EP 3 639 818 B1**
(45) Date of publication and mention of the grant of the patent: **07.07.2021**
(21) Application number: 19801189.2
(22) Date of filing: 27.05.2019
(51) Int. Cl.: A61K 31/131, A61K 31/5375, A61P 3/10, A61P 3/08, A61K 31/13, A61K 31/133, A61K 31/137

(54) **GLUCOSE CONSUMPTION PROMOTER AND GLYCOLYSIS PROMOTER**
GLUKOSEVERBRAUCHSPROMOTOR UND GLYKOLYSEPROMOTOR
PROMOTEUR DE CONSOMMATION DE GLUCOSE ET PROMOTEUR DE GLYCOLYSE

(30) Priority: 31.07.2018 JP 2018143056; 27.09.2018 JP 2018181302; 04.02.2019 WO PCT/JP2019/003915
(43) Date of publication of application: 22.04.2020
(73) Proprietor: Shinko Sangyo Co., Ltd., Takasaki-shi Gunma 370-0871 (JP); Sorimachi, Kenji, Takasaki-shi, Gunma 370-0041 (JP)
(72) Inventor: Sorimachi, Kenji, Takasaki-shi, Gunma 370-0041 (JP)
(74) Representative: J A Kemp LLP
(86) International application number: PCT/JP2019/020826
(87) International publication number: WO 2020/026570

(56) References cited:
- WO-A1-2014/080383
- WO-A1-2015/083164
- WO-A1-2017/046730
- WO-A2-2006/053043
- JP-A- H01 500 589

## Description

The present invention relates to a glucose consumption accelerator for use in the treatment or prevention of a disease caused by glucose concentration *in vivo,* specifically diabetes.

### BACKGROUND ART

Diabetes is a disease in which insulin does not work adequately, so that the glucose concentration (blood glucose level) in the blood continues to be high. It is known that long-term diabetes causes vascular damage due to high concentration of glucose, resulting in complications such as heart disease, blindness, renal failure, and amputation of the foot.

As a method for the treatment of diabetes, for example, there is known a method of controlling blood glucose level by replenishment of insulin from outside the body by insulin injection in the case of type 1 Diabetes, and by drug therapies such as oral hypoglycemic drugs and insulin injection, in addition to diet therapy and exercise therapy, in the case of type 2 Diabetes (Patent Literature 1). However, there is a need for new methods that can control glucose concentration.

Patent Literature 2 describes salts of Aramchol, including ethanolamine and diethanolamine salts. The salts are said to have improved physicochemical properties compared to Aramchol free acid and to be useful in the treatment of a disease or disorder associated with altered glucose metabolism.

### Citation List

### Patent Literature

### Patent Literature

Patent literature 1: JP 2015-205925 A
Patent literature 2: WO 2015/083164

### SUMMARY OF INVENTION

### Technical Problem

Accordingly, it is an object of the present invention to provide a new drug that can accelerate the consumption of glucose.

### Solution to Problem

In order to achieve the above object, the present invention provides:
[1] A glucose consumption accelerator comprising: at least one selected from the group consisting of 2-amino-1-cyclohexylethanol, 1-amino-2-propanol, 1,3-bis[tris(hydroxymethyl)methylamino]propane, N-cyclohexylethanolamine, dipropylamine, morpholine, propylamine, triethanolamine, triethylamine, and trishydroxymethylaminomethane for use in the treatment or prevention of diabetes.
[2] The glucose consumption accelerator for use according to [1], comprising: at least one selected from the group consisting of 2-amino-1-cyclohexylethanol, 1-amino-2-propanol, 1,3-bis[tris(hydroxymethyl)methylamino]propane, N-cyclohexylethanolamine, dipropylamine, morpholine, propylamine, triethanolamine, triethylamine, and trishydroxymethylaminomethane as a main component.
[3] The glucose consumption accelerator for use according to [1] or [2], further comprising: an oral additive.
[4] A compound selected from the group consisting of diethanolamine and diethylamine, for use in the treatment or prevention of diabetes.

The present invention also provides a pharmaceutical composition for use in treatment of diabetes, including: the glucose consumption accelerator as defined herein.

### Advantageous Effects of Invention

According to the present invention, a new drug that can accelerate the glucose consumption can be provided.

### BRIEF DESCRIPTION OF DRAWINGS

[FIGs. 1A and 1B] FIGs. 1A and 1B are graphs each showing relative values of glucose consumption of fibroblasts to which the drugs were added in Example 1.
[FIGs. 2A and 2B] FIGs. 2A and 2B are graphs each showing relative values of glucose consumption of fibroblasts to which the drugs of the comparative example were added in Example 1.
[FIGs. 3A and 3B] FIGs. 3A and 3B are graphs each showing relative values of glucose consumption of fibroblasts to which the drugs were added in Example 2.
[FIG. 4] FIG. 4 is a graph showing relative values of glucose consumption of fibroblasts to which the drugs were added in Example 3.
[FIG. 5] FIG. 5 is a graph showing relative values of glucose consumption of fibroblasts to which the drugs were added in Example 4.
[FIG. 6] FIG. 6 is a graph showing relative values of glucose consumption of liver cancer cells to which the drugs were added in Example 5.
[FIGs. 7A and 7B] FIGs. 7A and 7B are graphs each showing relative values of lactate concentration of fibroblasts and liver cancer cells to which the drugs were added in Example 6.
[FIGs. 8A and 8B] FIGs. 8A and 8B are graphs showing blood glucose levels of mice to which the drug was administered in Example 7.
[FIGs. 9A and 9B] FIGs. 9A and 9B are graphs each showing calculated values of AUC of the mice to which the drug was administered in Example 7.
[FIG. 10] FIG. 10 is a graph showing the lactate concentrations in the culture solution of each cell to which the drug was administered in Example 8.
[FIG. 11] FIG. 11 is a graph showing the lactate concentration in the culture solution of each cell to which the drug was administered in Example 9.

### DESCRIPTION OF EMBODIMENTS

The glucose consumption accelerator includes, for example, at least one selected from the group consisting of 2-amino-1-cyclohexylethanol, 1-amino-2-propanol, 1,3-bis[tris(hydroxymethyl)methylamino]propane, N-cyclohexylethanolamine, dipropylamine, morpholine, propylamine, triethanolamine, triethylamine, and trishydroxymethylaminomethane as a main component.

The glucose consumption accelerator of the present disclosure further include, for example, an oral additive.

The terms used in the present specification can be used in the meaning commonly used in the art unless otherwise specified.

The present invention is described below in details.

### (Glucose consumption accelerator)

As described above, the glucose consumption accelerator is characterized in that it contains at least one selected from the group consisting of 2-amino-1-cyclohexylethanol, 1-amino-2-propanol, 1,3-bis[tris(hydroxymethyl)methylamino]propane, N-cyclohexylethanolamine, dipropylamine, morpholine, propylamine, triethanolamine, triethylamine, and trishydroxymethylaminomethane (hereinafter also referred to as "drugs of the present disclosure"). In the glucose consumption accelerator of the present disclosure, other constitution and conditions are not particularly limited. In addition to the glucose consumption accelerator as described herein and defined in the claims, diethanolamine and diethylamine are also drugs of the present disclosure; the present invention further includes a compound selected from the group consisting of diethanolamine and diethylamine, for use in the treatment or prevention of diabetes.

The inventors of the present invention have found that these drugs accelerate glucose consumption and glycolysis, although the mechanism is unknown, thereby made the present invention.

In the present invention, the "glucose consumption" may be, for example, acceleration of a decrease in the glucose concentration or suppression of an increase in the glucose concentration. The "glucose consumption" may be measured, for example, by the method described in the examples below. The "glucose consumption" may be, for example, consumption of glucose by cells. In this case, the "glucose consumption" can also be referred to as, for example, "uptake of glucose into cells". Thus, the glucose consumption accelerator of the present disclosure can also be referred to as, for example, an uptake accelerator of glucose into cells. It is known that glucose is taken up, for example, via a glucose transporter present in a cell membrane. Thus, the glucose consumption accelerator of the present disclosure can also be referred to as, for example, an activator of a signaling cascade via a glucose transporter. However, the present invention is not limited thereto.

In the present invention, the "glycolysis" is, for example, a metabolic system starting from glucose. A metabolite of the glycolysis can be, for example, a lactate. Therefore, for example, as described below, by measuring the concentration of the lactate, the acceleration of the glycolysis can be examined. The glycolysis is also referred to as an anaerobic glycolysis, for example.

According to the glucose consumption accelerator of the present disclosure , as described above, the consumption of glucose can be accelerated. Thus, the glucose consumption accelerator of the present disclosure can be used as pharmaceutical compositions for use in the treatment of a disease caused by glucose concentration in vivo. The disease in the present invention is diabetes. In the present invention, "treatment" includes, for example, the meaning of prevention of the disease, the amelioration of the disease, and the amelioration of the prognosis of the disease, and may be any of them.

According to the glucose consumption accelerator of the present disclosure, for example, it is possible to accelerate the consumption of glucose and accelerate the glycolysis without suppressing the metabolism of lactate produced by the consumption of glucose.

The glucose consumption accelerator of the present disclosure may contain, for example, only one type of the drugs of the present disclosure or two or more types of them in combination as an active ingredient(s), and the number of types of the drugs to be contained is not particularly limited. The glucose consumption accelerator of the present disclosure includes the drug of the present disclosure, for example, as a main ingredient.

The glucose consumption accelerator of the present disclosure may be used, for example, *in vivo* or *in vitro.* The glucose consumption accelerator of the present disclosure can be used, for example, as a research reagent or, as described above and claimed, can be for use as a pharmaceutical.

The subject to be administered is not particularly limited. When the glucose consumption accelerator of the present disclosure is to be used *in vivo,* the subject to be administered is not particularly limited, and is, for example, a human or a non-human animal excluding a human. Examples of the non-human mammals include non-human animals such as mice, rats, rabbits, dogs, sheep, horses, cats, goats, monkeys, and guinea pigs. When the glucose consumption accelerator of the present disclosure is used *in vitro,* examples of the subject to be administered include cells, tissues, and organs. The cell may be, for example, cells collected from a living body or cultured cells. The cell is not particularly limited and examples thereof include fibroblasts, hepatocytes, and adipocytes.

The conditions of use of the glucose consumption accelerator of the present disclosure (hereinafter also referred to as "administration conditions") are not particularly limited, and for example, the administration form, administration timing, and dosage can be appropriately determined according to the type or the like of the subject to be administered. When the glucose consumption accelerator of the present disclosure is to be used *in vivo,* examples of the administration form include oral administration, intraperitoneal administration, and subcutaneous administration.

The dosage form of the glucose consumption accelerator of the present disclosure is not particularly limited, and can be appropriately determined according to the administration form, for example. In the case of oral administration, examples of the dosage form include capsules, extracts, elixirs, granules, pills, suspensions, fine granules, powders, spirits, tablets, syrups, dips and decoctions, tinctures, aromatics, lemonades, and stream extracts.

The glucose consumption accelerator of the present disclosure may contain, for example, an additive as required. When the glucose consumption accelerator of the present disclosure is used as a medicament, the additive is preferably a pharmaceutically acceptable additive. Examples of the additive include excipients, stabilizers, preservatives, buffers, corrigents, suspending agents, emulsifying agents, flavoring agents, dissolution assists, coloring agents, and viscosifiers. The additive may be, for example, an oral additive. Examples of the oral additive include caries preventive agents, intestinal regulators, and corrigents. In the present invention, the blending amount of the additive is not particularly limited, and it is only required not to hinder the function of the glucose consumption accelerator.

The conditions for administrating the glucose consumption accelerator of the present disclosure are not particularly limited, and, for example, the administration timing, administration period, and dosage can be appropriately determined according to the type, sex, age, site of the subject to be administered, for instance.

As a specific example, when the glucose consumption accelerator of the present disclosure is to be orally administered to a human, the total dosage per day is, for example, 100 to 5000 mg or 500 to 2500 mg. The number of times of administration per day is, for example, 1 to 5 times or 2 to 3 times.

### (Pharmaceutical composition)

The pharmaceutical composition for use in the treatment of diabetes according to the present invention is characterized in that it includes the glucose consumption accelerator, as described above. The pharmaceutical composition is characterized in that it includes the glucose consumption accelerator, and other constitution and conditions are not particularly limited. Regarding the pharmaceutical composition, reference can be made to the description as to the glucose consumption accelerator and the glycolysis accelerator. According to the pharmaceutical composition, for example, diabetes can be treated.

### (Glucose consumption accelerating method)

A glucose consumption accelerating method is characterized in that it includes a step of administering the glucose consumption accelerator to a subject to be administered. Such a method is characterized by including the step of administering the glucose consumption accelerator, and other steps and conditions are not particularly limited. The glucose consumption accelerator is as described above. The conditions for administrating the glucose consumption accelerator are not particularly limited, and, for example, are the same as those described in the description as to the glucose consumption accelerator.

### (Use of Drug)

The drug of the present disclosure is useful for accelerating glucose consumption and glycolysis, and for the treatment of diabetes.

### Examples

The examples of the present invention are described below. The present invention, however, is not limited by the following examples. The commercially available reagents were used based on the protocols thereof, unless otherwise specified.

### Example 1

The present example examined whether the drug has a glucose consumption accelerating effect on fibroblasts.

As drugs, 2-amino-1-cyclohexylethanol (Matrix Biochemicals), 2-aminoethanol (Tokyo Chemical Industry Co., Ltd.), 1-amino-2-propanol (Wako Pure Chemical Industries, Ltd.), 2-amino-1-phenylethanol (Tokyo Chemical Industry Co., Ltd.), 1,3-bis[tris(hydroxymethyl)-methylamino]propane (Tokyo Chemical Industry Co., Ltd.), N-cyclohexylethanolamine (Tokyo Chemical Industry Co., Ltd.), diethanolamine (Wako Pure Chemical Industries, Ltd.), diethylamine (Wako Pure Chemical Industries, Ltd.), dipropylamine (Tokyo Chemical Industry Co., Ltd.), HEPES sodium salt (MP Biomedicals), metoprolol tartrate (Tokyo Chemical Industry Co., Ltd.), morpholine (Wako Pure Chemical Co., Ltd.), octopamine (MP Biomedicals), propylamine (Tokyo Chemical Industry Co., Ltd.), triethylamine (Wako Pure Chemical Co., Ltd.), timolol maleate (Wako Pure Chemical Industries, Ltd.) and tris(hydroxymethyl)aminomethane (Wako Pure Chemical Industries, Ltd.) were used. Each drug was dissolved in distilled water to prepare a sample.

Rat-derived fibroblasts (Py-3Y1-S2, subculture strain) were then seeded in a 24-well microplate to achieve a density of 2×10⁵ cells/ml/well and cultured up to be monolayers. In the culture, DMEM (Nissui Pharmaceutical Co., Ltd.) was used as a culture medium. To this culture solution, the samples were added so that each drug had a final concentration of 0.5 mg/ml, and further cultured for 12 to 24 hours. As a control of the drug, the culture was performed in the same manner except that distilled water to which the drug was not added was added to the culture solution instead of the sample.

After the addition of the drug, the glucose concentration of each of the culture solutions was measured immediately after the start of the culture and after the completion of the culture. The glucose concentration was measured using a glucose assay kit (Waco). Then, the glucose concentration after the completion of the culture was divided by the glucose concentration immediately after the start of the culture (5.6 mmol per liter) to calculate the glucose consumption. Further, the glucose consumption in the control was set as a reference value of 100, and the relative value of the glucose consumption in each sample was calculated.

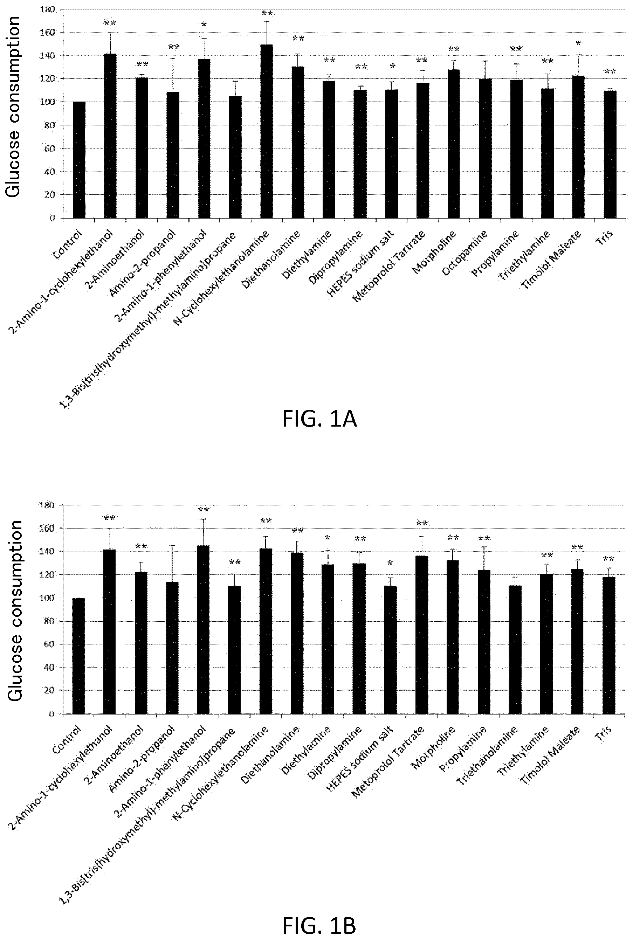

The results are shown in FIG. 1A. FIG. 1A is a graph showing the relative values of the glucose consumption of fibroblasts to which the above-described drugs each having a concentration of 0.5 mg/ml were added. In FIG. 1A, the vertical axis represents the relative value of glucose consumption, and the horizontal axis represents the drug. As shown in FIG. 1A, in every sample, the addition of each drug resulted in a relative glucose consumption value of 100 or more, and an increase in the glucose consumption. Among them, the addition of 2-amino-1-cyclohexylethanol, 2-aminoethanol, 1-amino-2-propanol, 2-amino-1-phenylethanol, N-cyclohexylethanolamine, diethanolamine, diethylamine, dipropylamine, HEPES sodium salt, metoprolol tartrate, morpholine, propylamine, triethylamine, timolol maleate, and tris(hydroxymethyl)aminomethane significantly increased glucose consumption compared to a control (t-test: p < 0.05 or p < 0.1, indicated by "**" or "*" in FIG. 1A), which showed strong glucose consumption accelerating effect.

Furthermore, the glucose consumption accelerating effect was examined under the same conditions as described above except that 2-amino-1-cyclohexylethanol, 2-aminoethanol, 1-amino-2-propanol, 2-amino-1-phenylethanol, 1,3-bis[tris(hydroxymethyl)-methylamino]propane, N-cyclohexylethanolamine, diethanolamine, diethylamine, dipropylamine, HEPES sodium salt, metoprolol tartrate, morpholine, propylamine, triethanolamine (Tokyo Chemical Industry Co., Ltd.), triethylamine, timolol maleate, and tris(hydroxymethyl)aminomethane were used as drugs and each drug had a final concentration of 1 mg/ml.

The results are shown in FIG. 1B. FIG. 1B is a graph showing the relative values of the glucose consumption of fibroblasts to which the above-described drugs each having a concentration of 1 mg/ml were added. In FIG. 1B, the vertical axis represents the relative value of glucose consumption, and the horizontal axis represents the drug. As shown in FIG. 1B, in every sample, the addition of each drug resulted in a relative glucose consumption value of 100 or more, and an increase in the glucose consumption. Among them, the addition of 2-Amino-1-cyclohexylethanol, 2-Aminoethanol, 2-Amino-1-phenylethanol, 1,3-Bis[tris(hydroxymethyl)-methylamino]propane, N-Cyclohexylethanolamine, Diethanolamine, Diethylamine, Dipropylamine, HEPES sodium salt, Metoprolol Tartrate, Morpholine, Propylamine, Triethylamine, Timolol maleate, and tris(hydroxymethyl)aminomethane significantly increased glucose consumption compared to a control (t-test: p < 0.05 or p < 0.1, indicated by "**" or "*" in FIG. 1B), which showed strong glucose consumption accelerating effect.

Next, as a comparative example, the glucose consumption accelerating effect was examined under the same conditions as described above except that tricine and trimethylolpropane, which are drugs having no structures represented by the chemical formula (1), were used.

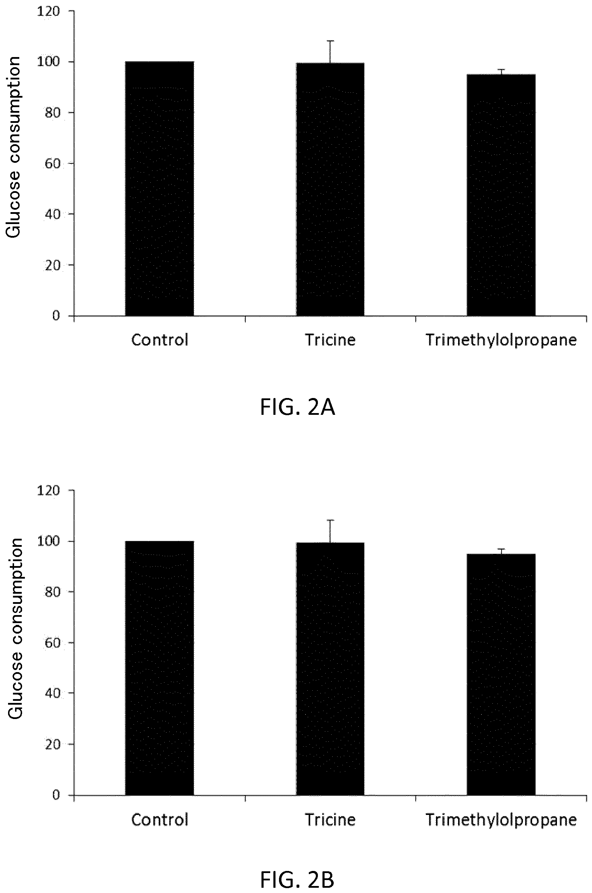

The results are shown in FIGs. 2A and 2B. FIGs. 2A and 2B are graphs each showing the relative values of the glucose consumption of fibroblasts to which the above-described drugs of the comparative example were added. FIG. 2A shows the result of the case in which each drug of the comparative example had a concentration of 0.5 mg/ml, and FIG. 1B shows the result of the case in which each drug of the comparative example had a concentration of 1 mg/ml. In FIGs. 2A and 2B, the vertical axis represents the relative value of the glucose consumption, and the horizontal axis represents the drug. As shown in FIGs. 2A and 2B, the addition of each drug of the comparative example having a concentration of 0.5 mg/ml or 1 mg/ml resulted in a relative glucose consumption value of less than 100 and no increase in glucose consumption in any of the samples.

The results shown in FIGs. 1A to FIG. 2B showed that the drugs of the present disclosure exhibited the glucose consumption accelerating effect by having the structure represented by the chemical formula (1).

### Example 2

The present example examined that the drug of the present disclosure has a glucose consumption accelerating effect on fibroblasts in a dose dependent manner.

Samples of 2-amino-1-cyclohexylethanol and 2-amino-1-phenylethanol, each of which exhibited a strong glucose consumption accelerating effect in Example 1, were prepared. The fibroblasts were cultured under the same conditions as in Example 1. To this culture solution, the samples were added so that each drug had final concentrations of 0.1, 0.25, and 0.5 mg/ml. The relative value of the glucose consumption in each sample was calculated in the same manner as in Example 1.

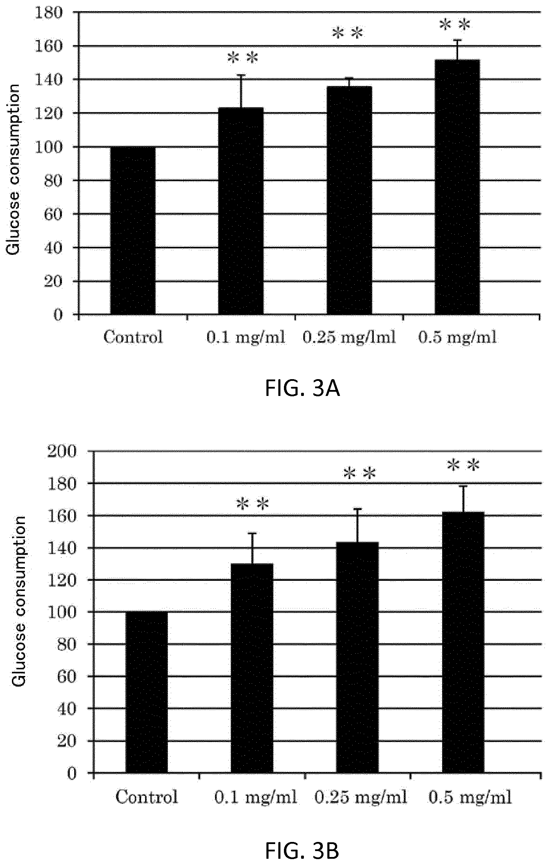

The results are shown in FIGs. 3A and 3B. FIGs. 3A and 3B are graphs each showing the relative values of the glucose consumption of fibroblasts to which the above-described drugs were added. FIG. 3A shows the result of the case of adding 2-amino-1-cyclohexylethanol, and FIG. 3B shows the result of the case of adding 2-amino-1-phenylethanol. In FIGs. 3A and 3B, the vertical axis represents the relative value of the glucose consumption, and the horizontal axis represents the concentration of each drug. As shown in FIG. 3A, the addition of 0.1, 0.25, and 0.5 mg/ml 2-amino-1-cyclohexylethanol to fibroblasts increased glucose consumption in a dose-dependent manner up to about 120, about 130, and about 150, respectively. The glucose concentration was significantly increased compared to the control (t-test: p < 0.05, indicated by "**" in FIGs. 3A and 3B) at any concentration, which showed a strong glucose consumption accelerating effect. Furthermore, as shown in FIG. 3B, the addition of 0.1, 0.25, and 0.5 mg/ml 2-amino-1-phenylethanol increased glucose consumption in a dose-dependent manner up to about 130, about 140, and about 160, respectively. The glucose concentration was significantly increased compared to the control, which showed a strong glucose consumption accelerating effect.

As shown in each graph of FIG. 3A and 3B, all of the administered drugs exhibited glucose consumption accelerating effects in a dose dependent manner. In addition, it was found that the glucose consumption accelerating effect was effectively obtained even at a low dose.

### Example 3

The present example examined that the drug of the present disclosure has a glucose consumption accelerating effect on fibroblasts in which glucose consumption was suppressed.

First, samples of streptozotocin (STZ), alloxan, and nicotinamide were prepared in the same manner as in Example 1. The fibroblasts were cultured. To this culture solution, the samples were added so that each drug had a final concentration 1 mg/ml. Streptozotocin, alloxan, and nicotinamide are drugs all known to produce symptoms of diabetes when administered to the body. The relative value of the glucose consumption was calculated in the same manner as in Example 1.

Next, samples of streptozotocin, alloxan, and nicotinamide plus 2-amino-1-phenylethanol (2-A-1-P) were prepared in the same manner. The concentration of 2-A-1-P was 1 mg/ml. The relative value of the glucose consumption was calculated in the same manner.

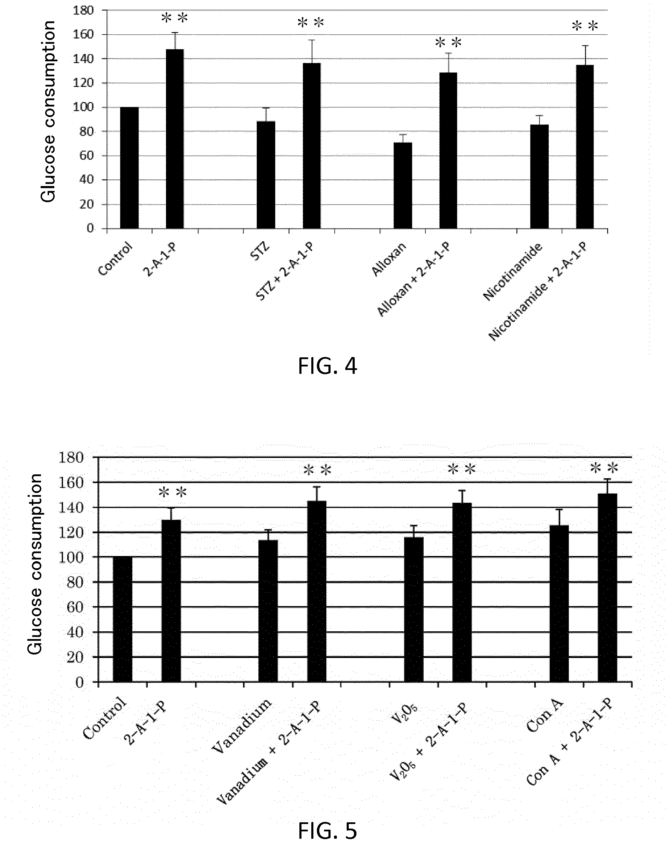

The results are shown in FIG. 4. FIG. 4 is a graph showing the relative values of the glucose consumption of fibroblasts to which the above-described drugs were added. In FIG. 4, the vertical axis represents the relative value of the glucose consumption, and the horizontal axis represents the drug. As shown in FIG. 4, the addition of 1 mg/ml streptozotocin, alloxan, and nicotinamide resulted in glucose consumption relative values of about 90, about 70, and about 85, respectively, and a decrease in glucose consumption. On the other hand, the addition of 2-A-1-P in addition to streptozotocin, alloxan, and nicotinamide resulted in a glucose consumption relative value of 100 or more, and recovery of the glucose consumption. This showed that 2-amino-1-phenylethanol exhibited the glucose consumption accelerating effect also on fibroblasts in which glucose consumption was suppressed.

### Example 4

The present example examined that the drug of the present disclosure has further glucose consumption accelerating effect on fibroblasts in which glucose consumption was accelerated.

First, samples of vanadium, V₂O₅, and concanavalin A (ConA) were prepared in the same manner as in Example 1. The fibroblasts were cultured. To this culture solution, the samples were added so that vanadium had a final concentration of 1.0 mg/ml and ConA had a final concentration of 100 µg/ml. Vanadium, V₂O₅, and concanavalin A are drugs all known to exhibit insulin-like effects upon administration to fibroblasts. The relative value of the glucose consumption in each sample was calculated in the same manner as in Example 1.

Next, samples of vanadium, V₂O₅, and concanavalin A plus 2-amino-1-phenylethanol (2-A-1-P) were prepared in the same manner. The concentration of 2-A-1-P was 0.5 mg/ml. The relative value of the glucose consumption was calculated in the same manner.

The results are shown in FIG. 5. FIG. 5 is a graph showing the relative values of the glucose consumption of fibroblasts to which the above-described drugs were added. In FIG. 5, the vertical axis represents the relative value of the glucose consumption, and the horizontal axis represents the drug. As shown in FIG. 5, the addition of vanadium, V₂O₅, and concanavalin A resulted in glucose consumption relative values of about 110, about 115, and about 120, respectively, and an increase in glucose consumption. On the other hand, the addition of 2-A-1-P in addition to vanadium, V₂O₅ and concanavalin A resulted in glucose consumption relative values of about 145, about 140, and about 150, respectively, and a further increase in the glucose consumption. This showed that 2-amino-1-phenylethanol exhibited the glucose consumption accelerating effect also on fibroblasts in which glucose consumption was accelerated by vanadium, V₂O₅ and concanavalin A.

### Example 5

The present example examined that the drug of the present disclosure has a glucose consumption accelerating effect on liver cancer cells.

First, samples of 2-amino-1-cyclohexylethanol and 2-amino-1-phenylethanol were prepared in the same manner as in Example 1. Instead of the rat-derived fibroblasts (Py-3Y1-S2, subculture strain), rat-derived liver cancer cells (Ry121B, subculture line) were used, and the rat-derived liver cancer cells were cultured under the same conditions as in Example 1. To this culture solution, the samples were added so that each drug had a final concentration of 1 mg/ml. As a control, the culture was performed in the same manner except that distilled water to which the drug was not added was added to the culture solution instead of the sample. The relative value of the glucose consumption in each sample was calculated in the same manner as in Example 1.

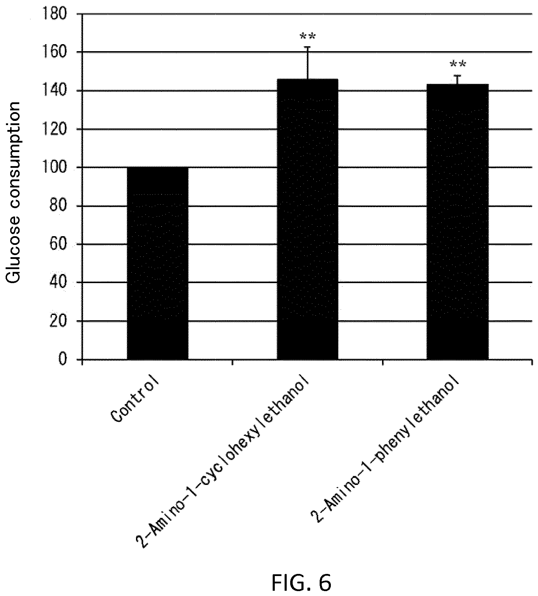

The results are shown in FIG. 6. FIG. 6 is a graph showing the relative values of the glucose consumption of liver cancer cells to which the above-described drugs were added. In FIG. 6, the vertical axis represents the relative value of the glucose consumption, and the horizontal axis represents the drug.

As shown in FIG. 6, also in liver cancer cells, the addition of 2-amino-1-cyclohexylethanol and 2-amino-1-phenylethanol also resulted in glucose consumption relative values of about 145 and about 140, respectively, and a significant increase in glucose consumption compared to the control.

The results shown in FIG. 6 showed that the drug of the present disclosure exhibited the glucose consumption accelerating effect also on liver cancer cells.

### Example 6

The present example examined that the glucose consumption accelerating effect by the drug of the present disclosure accelerates the glycolysis by cells.

First, samples of 2-amino-1-cyclohexylethanol and 2-amino-1-phenylethanol were prepared in the same manner as in Example 1. The fibroblasts and the liver cancer cells were cultured under the same conditions as in Examples 1 and 5. To these culture solutions, the samples were added so that each drug had a final concentration of 1 mg/ml. As a control, the culture was performed in the same manner except that distilled water to which the drug was not added was added to the culture solution instead of the sample.

After the addition of the drug, the culture solution was diluted so that the weight ratio of the culture solution to distilled water was 1:19, and the lactate concentration of the diluted culture solution was measured. The lactate concentration was measured using a lactate assay kit (product name: Lactate Assay Kit-WST, product of Dojindo Laboratories). The lactate concentration in the control was set as a reference value of 100, and the relative value of the lactate concentration in each sample was calculated.

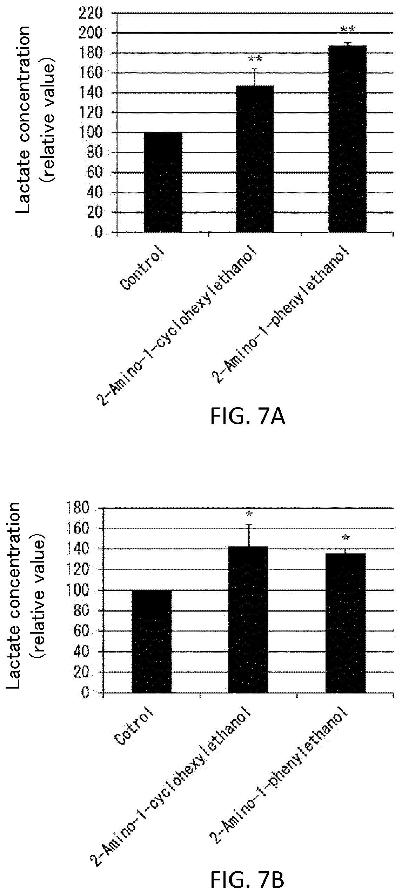

The results are shown in FIGs. 7A and 7B. FIGs. 7A and 7B are graphs each showing the relative values of the lactate concentration of the cells to which the drugs were added. FIG. 7A shows the results of fibroblasts, and FIG. 7B shows the results of liver cancer cells. In FIGs. 7A and 7B, the vertical axis represents the relative value of the lactate concentration, and the horizontal axis represents the drug.

As shown in FIG. 7A, in fibroblasts, the addition of 2-amino-1-cyclohexylethanol and 2-amino-1-phenylethanol increased the lactate concentration up to about 145 and about 185, respectively. As shown in FIG. 7B, in liver cancer cells, the addition of 2-amino-1-cyclohexylethanol and 2-amino-1-phenylethanol increased the lactate concentration up to about 140 and about 135, respectively.

The results shown in FIGs. 7A and 7B showed that the drug of the present disclosure increased the lactate concentration in fibroblasts and liver cancer cells, and exhibited the glucose consumption accelerating effect.

### Example 7

The present example examined that the drug of the present disclosure lowers the blood glucose level of mice.

As a drug, 2-amino-1-phenylethanol was used. First, glucose (KANTO CHEMICAL CO., INC.) was dissolved in water for injection so as to achieve a concentration of 200 mg/ml, thereby preparing a glucose solution. Next, the drug was dissolved in the water for injection, and then the glucose solution was added thereto so that the drug and glucose had final concentrations of 2.5 mg/ml and 100 mg/ml, respectively, thereby preparing a sample. As a control of the drug, the culture was performed in the same manner except that 100 mg/ml glucose solution to which the drug was not added was used instead of the sample.

Male mice of 6-week-old ICR strain were purchased from Japan SLC, Inc., and preliminary rearing was carried out for about 1 week to check there was no abnormality in performance status. The conditions for the preliminary rearing were as follows: 4 mice each were housed in a polycarbonate cage, room temperature was 23°C±3°C, and illumination time was 12 hours/day. Diets (mice and rat chow, product of Nosan Corporation.) and drinking water (tap water) were freely ingested.

After the preliminary rearing, the mice were fasted for about 21 hours, and then body weight and blood glucose levels were measured. The mice were divided into the total of two groups, namely, a test group and a control group to ensure that there was no variation in blood glucose levels between the groups. The number of animals in each group was eight. The table 2 shows the body weight (g) of the mice at the time of grouping.

**[Table 2]**

| Group | Test animal | Body weight (g) |
|---|---|---|
| Control group | No. 1 | 30.9 |
| | No. 2 | 29.6 |
| | No. 3 | 29.9 |
| | No. 4 | 31.0 |
| | No. 5 | 27.7 |
| | No. 6 | 31.4 |
| | No. 7 | 31.5 |
| | No. 8 | 29.6 |
| Test group | No. 1 | 29. 2 |
| | Mo. 2 | 32.3 |
| | No. 3 | 30.8 |
| | No. 4 | 30.2 |
| | No. 5 | 28.4 |
| | No. 6 | 28.0 |
| | No. 7 | 29.4 |
| | No. 8 | 30.4 |

After the grouping, mice of the test group and the control group were orally administered a single dose of the sample and the control, respectively, using a gastric sonde to a dose volume of 20 ml/kg. That is, the test group mice were administered the drug and glucose at doses of 50 mg/kg and 2000 mg/kg, respectively, and the control group mice were administered glucose at a dose of 2000 mg/kg. With the time of the administration being regarded as 0 minutes, the blood glucose level was measured at 30 minutes, 60 minutes, 90 minutes, and 120 minutes. The blood glucose level was measured by using an ACCU-CHEK Aviva (Roche Diagnostics K.K.), blood was collected by inserting an injection needle into the tip of the tail, and the blood glucose level was measured for the collected blood.

As to the blood glucose levels at 0 minutes, 30 minutes, 60 minutes, 90 minutes and 120 minutes, the test group and the control group were compared by t-test. The significance levels were 5% and 1%.

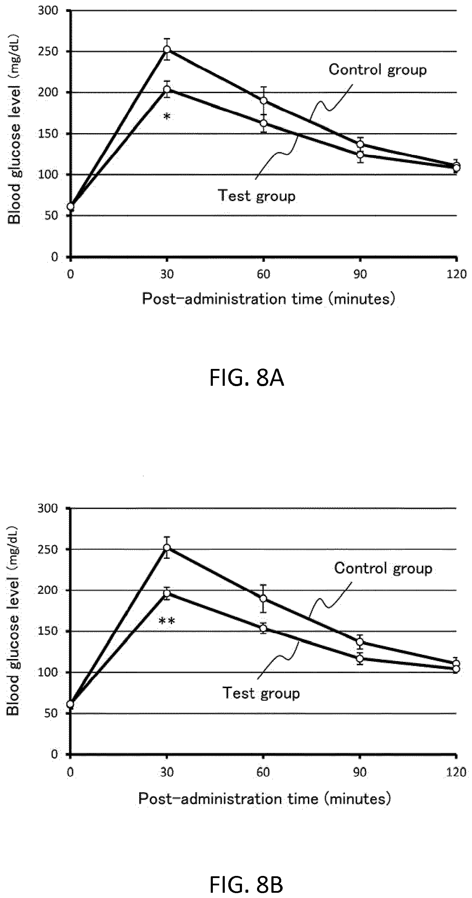

The results are shown in the table 3 and FIGs. 8A and 8B. The table 3 shows the measured values (mg/dL) of the blood glucose levels in the individuals at the predetermined time after the administration, and FIGs. 8A and 8B are graphs each showing the mean values of the blood glucose levels in the test group and the control group at the predetermined time after the administration. In FIGs. 8A and 8B, the vertical axis represents the blood glucose level (mg/dL), and the horizontal axis represents the post-administration time (minutes). In FIG. 8A, the mean value was calculated based on the blood glucose levels obtained for all the individuals (No. 1 to No. 8) in the control group and the test group. On the other hand, as shown in the table 3, the blood glucose level after glucose administration of one individual (No. 8) in the test group showed abnormal values compared to the calculated values of the other seven individuals (No. 1 to No. 7) in the test group. Therefore, in FIG. 8B, the data of one individual (No. 8) in the test group showing the abnormal values was excluded, and the mean value was calculated based on the blood glucose levels obtained for the other seven individuals (No. 1 to No. 7) in the test group.

**[Table 3]**

| | | Post-administration time (minutes) | | | | |
|---|---|---|---|---|---|---|
| Group | Test animal | 0 | 30 | 60 | 90 | 120 |
| Control group | No. 1 | 41 | 237 | 166 | 123 | 90 |
| | No. 2 | 47 | 294 | 231 | 138 | 107 |
| | No. 3 | 55 | 193 | 147 | 123 | 92 |
| | No. 4 | 53 | 268 | 268 | 189 | 149 |
| | No. 5 | 81 | 309 | 221 | 144 | 134 |
| | No. 6 | 67 | 251 | 155 | 125 | 100 |
| | No. 7 | 81 | 233 | 130 | 114 | 104 |
| | No. 8 | 60 | 235 | 201 | 140 | 110 |
| Test group | No. 1 | 46 | 177 | 151 | 121 | 116 |
| | No. 2 | 53 | 194 | 136 | 93 | 91 |
| | No. 3 | 61 | 194 | 180 | 138 | 112 |
| | No. 4 | 70 | 222 | 171 | 138 | 113 |
| | No. 5 | 54 | 165 | 136 | 117 | 108 |
| | No. 6 | 60 | 202 | 141 | 91 | 79 |
| | No. 7 | 86 | 219 | 160 | 120 | 111 |
| | No. 8 | 59 | 256 | 223 | 176 | 134 |

As shown in FIGs. 8A and 8B, the test group tended to have lower blood glucose levels at 30, 60, 90 and 120 minutes after administration compared to the control group. In particular, at 30 minutes after administration, the test group had significantly lower blood glucose levels (P < 0.05, and P < 0.01) compared to the control group.

In addition, the area under the curves (AUCs) were calculated for the measured blood glucose levels. The AUC was calculated by calculating the area surrounded by the measurement curve and a straight line parallel to the time axis and passing through the measured value at the time of administration in the graph with the measured value on the vertical axis and the time on the horizontal axis. Then, the test group and the control group were compared by t-test for AUC in the same manner as the comparison for the blood glucose level.

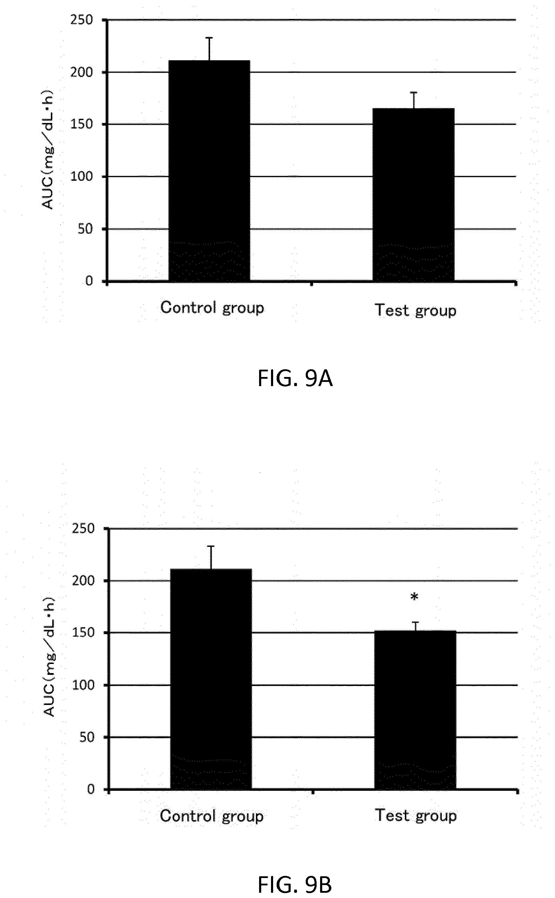

The results are shown in the table 4 and FIGs. 9A and 9B. The table 4 shows the calculated values of AUC (mg/dL·h) in the individuals, and FIGs. 9A and 9B are graphs each showing the mean values of AUC in the test group and the control group. In FIGs. 9A and 9B, the vertical axis represents AUC (mg/dL·h), and the horizontal axis represents the control group and the test group. In FIG. 9A, the mean value was calculated based on the calculated values of AUC obtained for all the individuals (No. 1 to No. 8) in the control group and the test group. On the other hand, as shown in Table 4, the calculated value (258) of AUC of one individual (No. 8) in the test group showed an abnormal value compared to the calculated values of the other seven individuals (No. 1 to No. 7) in the test group. Therefore, in FIG. 9B, the data of one individual (No. 8) in the test group showing the abnormal value was excluded, and the mean value was calculated based on the calculated values of AUC obtained for the other seven individuals (No. 1 to No. 7) in the test group.

**[Table 4]**

| | | AUC | |
|---|---|---|---|
| Group | Test animal | Calculated value | Mean value ± Standard error |
| Control group | No.1 | 214 | 211±21 |
| | No.2 | 276 | |
| | No.3 | 158 | |
| | No.4 | 307 | |
| | No.5 | 229 | |
| | No.6 | 173 | |
| | No.7 | 123 | |
| | No.8 | 211 | |
| Test group | No.1 | 173 | 165±15 |
| | No.2 | 142 | |
| | No.3 | 177 | |
| | No.4 | 171 | |
| | No.5 | 142 | |
| | No.6 | 132 | |
| | No.7 | 127 | |
| | No.8 | 258 | |

As shown in FIG. 9A, the test group tended to have lower AUC values compared to the control group. As shown in FIG. 9B, the test group had significantly lower AUC value (P < 0.05) compared to the control group.

The results shown in FIGs. 8A to 9B showed that the drug of the present disclosure lowered the blood glucose level of mice.

### Example 8

The present example examined that the drug of the present disclosure exhibits a lactate synthesis accelerating effect even after long-term culture.

First, a sample of 2-amino-1-cyclohexylethanol (2-amino-1-cyclohexyl EOH) was prepared in the same manner as in Example 1. In addition to the rat-derived fibroblasts (Py-3Y1-S2, subculture line), human-derived esophageal cancer cells (TE-13, subculture line), African green monkey-derived kidney epithelial cells (VERO, subculture line), and human-derived hepatocellular cancer cells (HepG2, subculture line) were used and cultured under the same conditions as in Example 1. To these culture solutions, the sample was added so that the drug had a final concentration of 0.5 mg/ml. Thereafter, the culture solutions were further cultured for 24 to 48 hours. The culture time after the addition of the sample was set to the same condition for each cell. As a control 1, the culture was performed in the same manner except that distilled water to which the drug was not added was added to the culture solution instead of the sample. As a control 2, a sample of biguanide was prepared in the same manner as in Example 1, and the culture was performed in the same manner as in Example 1 except that the sample of biguanide was added instead of the sample. Then, the lactate concentration in each sample was measured in the same manner as in Example 6. The total of five experiments (one for Py-3Y1-S2, TE-13 and HepG2 and two for VERO) were performed using the four types of cells.

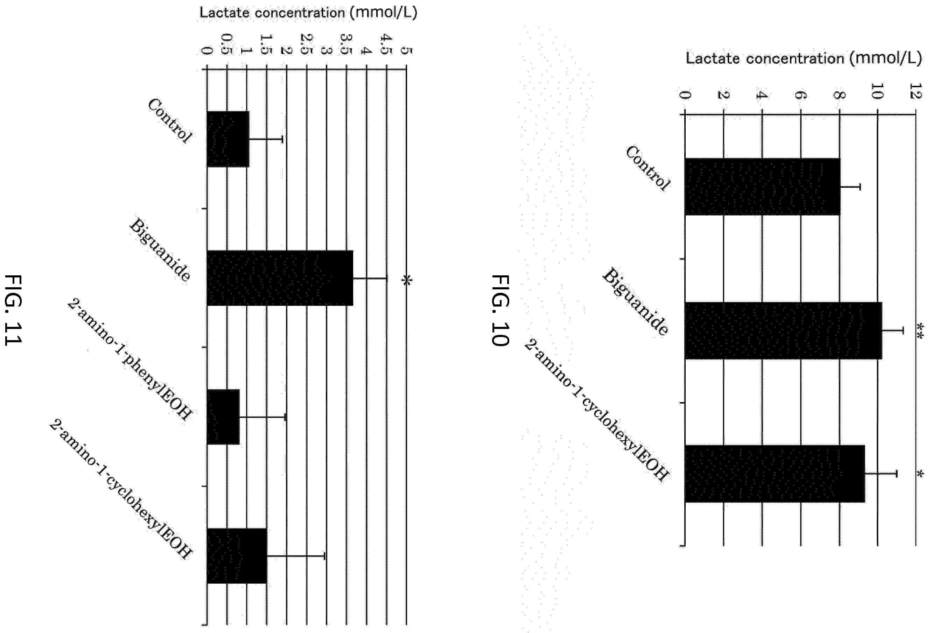

The results are shown in FIG. 10. FIG. 10 is a graph showing the lactate concentration in the culture solution of each cell to which the drugs were added. The value shown in the graph represents the mean value of the results of the five experiments. In FIG. 10, the vertical axis represents the lactate concentration (mmol/L) in the culture solution, and the horizontal axis represents the drug. As shown in FIG. 10, when 24 to 48 hours culture was performed after adding 0.5 mg/ml 2-amino-1-cyclohexylethanol, the lactate concentration was 9.35 mmol/L and the lactate concentration was increased compared to control 1 (Control) (P < 0.05). Also in control 2 (Biguanide), the addition of biguanide increased the lactate concentration (P < 0.01). This showed that 2-amino-1-cyclohexylethanol increased the lactate concentration even after long-term culture, and exhibited the glycolysis accelerating effect.

It seems that most of the glucose in the culture solution is changed to lactate after 24 to 48 hours culture. For example, since two molecules of lactate are synthesized from one molecule of glucose, if all of the glucose (5.6 mmol/L) immediately after the start of the culture is changed to lactate, the lactate concentration in the culture solution becomes 11.2 mmol/L. As shown in FIG. 10, when 24 to 48 hours culture was performed after adding each sample, the lactate concentration in the culture solution was about 8 to 10 mmol/L. This shows that, for example, about 70 to 90% of glucose was consumed and changed to lactate by culturing for 24 to 48 hours after addition of each sample. This also shows that 2-amino-1-cyclohexylethanol exhibits a glucose consumption accelerating effect even in the presence of, for example, a low concentration of glucose. The present invention, however, is not limited thereto.

### Example 9

The present example examined that the drug of the present disclosure does not suppress lactate metabolism.

First, samples of 2-amino-1-cyclohexylethanol (2-amino-1-cyclohexyl EOH) and 2-amino-1-phenylethanol (2-amino-1-phenyl EOH) were prepared in the same manner as in Example 1. Four types of cells were used as in Example 8, and each of the cells was cultured under the same conditions as in Example 1. To these culture solutions, the samples were added so that the drug had a final concentration of 0.5 mg/ml. Thereafter, the culture time was lengthened to 2 to 3 days (48 to 72 hours) compared to Example 8. As a control 1, the culture was performed in the same manner except that distilled water to which the drug was not added was added to the culture solution instead of the sample. As a control 2, the culture was performed in the same manner as the control 2 except that a sample of biguanide prepared in the same manner as in Example 1 was added to the culture solution instead of the sample. Then, the lactate concentration in each sample was measured in the same manner as in Example 6. Each of the four cells was used for a total of four experiments.

The results are shown in FIG. 11. FIG. 11 is a graph showing the lactate concentration of each cell to which the drug was added. The value shown in the graph represents the mean value of the results of the four experiments. In FIG. 11, the vertical axis represents the lactate concentration (mmol/L) in the culture solution, and the horizontal axis represents the drug. As shown in FIG. 11, when 2 to 3 days culture was performed after addition of 0.5 mg/ml 2-amino-1-cyclohexylethanol and 2-amino-1-phenylethanol, the lactate concentrations were equivalent to that of control 1 (Control). On the other hand, in Control 2 (biguanide), when 2 to 3 days culture was performed after addition of biguanide, the lactate concentration was about 3.5 times (P < 0.05) compared to Control 1. It is known that the lactate in the culture solution produced as a metabolite of the glycolysis is subsequently metabolized. Thus, since the lactate was metabolized to the same extent as control 1, it was examined that 2-amino-1-cyclohexylethanol and 2-amino-1-phenylethanol did not suppress the metabolism of lactate. In contrast, it was examined that biguanide suppressed the metabolism of lactate.

### Industrial Applicability

As described above, according to the present invention, glucose consumption can be accelerated by including at least one selected from the group consisting of 2-amino-1-cyclohexylethanol, 1-amino-2-propanol, 1,3-bis[tris(hydroxymethyl)methylamino]propane, N-cyclohexylethanolamine, diethanolamine, diethylamine, dipropylamine, morpholine, propylamine, triethanolamine, triethylamine, and trishydroxymethylaminomethane. Since the drug can accelerate the glucose consumption in this manner, for example, the drug can be used as a therapeutic agent for diabetes. Therefore, the present invention is extremely useful in the field of medicine.

## Claims

1. A glucose consumption accelerator comprising:
at least one selected from the group consisting of 2-amino-1-cyclohexylethanol, 1-amino-2-propanol, 1,3 -bis[tris(hydroxymethyl)methylamino]propane, N-cyclohexylethanolamine, dipropylamine, morpholine, propylamine, triethanolamine, triethylamine, and
trishydroxymethylaminomethane; for use in the treatment or prevention of diabetes.

2. The glucose consumption accelerator for use according to claim 1, comprising:
at least one selected from the group consisting of 2-amino-1-cyclohexylethanol, 1-amino-2-propanol, 1,3 -bis[tris(hydroxymethyl)methylamino]propane, N-cyclohexylethanolamine, dipropylamine, morpholine, propylamine, triethanolamine, triethylamine, and
trishydroxymethylaminomethane as a main component.

3. The glucose consumption accelerator for use according to claim 1 or 2, further comprising:
an oral additive.

4. A compound selected from the group consisting of diethanolamine and diethylamine, for use in the treatment or prevention of diabetes.

## Patentansprüche

1. Glukoseverbrauchsbeschleuniger, umfassend:
mindestens eines, ausgewählt aus der Gruppe bestehend aus 2-Amino-1-cyclohexylethanol, l-Amino-2-propanol, 1,3-bis[tris(Hydroxymethyl)methylamino]propan, N-Cyclohexylethanolamin, Dipropylamin, Morpholin, Propylamin, Triethanolamin, Triethylamin und Trishydroxymethylaminomethan; zur Verwendung bei der Behandlung oder Prävention von Diabetes.

2. Glukoseverbrauchsbeschleuniger zur Verwendung nach Anspruch 1, umfassend:
mindestens eines, ausgewählt aus der Gruppe bestehend aus 2-Amino-1-cyclohexylethanol, l-Amino-2-propanol, 1,3-bis[tris(Hydroxymethyl)methylamino]propan, N-Cyclohexylethanolamin, Dipropylamin, Morpholin, Propylamin, Triethanolamin, Triethylamin und Trishydroxymethylaminomethan als eine Hauptkomponente.

3. Glukoseverbrauchsbeschleuniger zur Verwendung nach Anspruch 1 oder 2, ferner umfassend:
ein orales Additiv.

4. Verbindung, ausgewählt aus der Gruppe bestehend aus Diethanolamin und Diethylamin, zur Verwendung bei der Behandlung oder Prävention von Diabetes.

## Revendications

1. Accélérateur de consommation de glucose comprenant :
au moins un élément choisi dans le groupe constitué par le 2-amino-1-1cyclohexyléthanol, le 1-amino-2-propanol, le 1,3-bis[tris(hydroxyméthyl)méthylamino]propane, 1a N-cyclohexyléthanolamine, la dipropylamine, la morpholine, la propylamine, la triéthanolamine, la triéthylamine, et le trishydroxyméthylaminométhane ; pour une utilisation dans le traitement ou la prévention du diabète.

2. Accélérateur de consommation de glucose pour une utilisation selon la revendication 1, comprenant :
au moins un élément choisi dans le groupe constitué par le 2-amino-1-1cyclohexyléthanol, le 1-amino-2-propanol, le 1,3-bis[tris(hydroxyméthyl)méthylamino]propane, 1a N-cyclohexyléthanolamine, la dipropylamine, la morpholine, la propylamine, la triéthanolamine, la triéthylamine, et le trishydroxyméthylaminométhane en tant que composant principal.

3. Accélérateur de consommation de glucose pour une utilisation selon la revendication 1 ou 2, comprenant en outre :
un additif oral.

4. Composé choisi dans le groupe constitué par la diéthanolamine et la diéthylamine, pour une utilisation dans le traitement ou la prévention du diabète.
